# EUROPEAN PATENT SPECIFICATION

(11) **EP 4 272 690 B1**
(45) Date of publication and mention of the grant of the patent: **05.11.2025**
(21) Application number: 21913234.7
(22) Date of filing: 16.09.2021
(51) Int. Cl.: A61C 7/00

(54) **ORTHODONTIC TREATMENT SYSTEM AND DESIGN METHOD THEREOF**
ORTHODONTISCHES BEHANDLUNGSSYSTEM SOWIE ENTWURFSVERFAHREN DAFÜR
SYSTÈME DE TRAITEMENT ORTHODONTIQUE ET SON PROCÉDÉ DE CONCEPTION

(30) Priority: 31.12.2020 CN 202023325100 U; 31.12.2020 CN 202011638977
(43) Date of publication of application: 08.11.2023
(73) Proprietor: Shanghai Smartee Denti-Technology Co., Ltd., Shanghai 201210 (CN)
(72) Inventor: WANG, Xingxing, Shanghai 200120 (CN); WU, Gang, Shanghai 200120 (CN); YAO, Junfeng, Shanghai 200120 (CN)
(74) Representative: Balder IP Law, S.L.
(86) International application number: PCT/CN2021/118853
(87) International publication number: WO 2022/142487

(56) References cited:
- WO-A1-2009/017826
- CN-A- 1 655 732
- CN-A- 1 655 733
- CN-A- 105 266 905
- CN-A- 105 434 062
- CN-A- 107 106 258
- CN-A- 112 674 890
- CN-A- 112 842 573
- CN-U- 210 931 951
- CN-U- 211 094 852
- US-A1- 2009 311 645
- US-A1- 2016 135 924
- US-A1- 2018 360 567
- US-A1- 2020 085 548

## Description

### TECHNICAL FIELD

The present application relates to the field of medical devices and in particular, to the field of invisible orthodontics, and more particularly to an orthodontic system, a design method thereof, and a method for preparing an orthodontic system, applied to the orthodontic treatment of adolescents during a period of tooth replacement.

### BACKGROUND

Invisible aligners are recognized by many consumers for their aesthetics, wearing comfort and good orthodontic effect. Among them, adolescents are a special part of the cases, for whom there is a certain stage in which deciduous teeth fall out and permanent teeth erupt, which is different from the adult permanent teeth orthodontic treatment. In a process of orthodontic treatment with the invisible aligner, in response to the teeth being treated according to the same structure as the actual model in the mouth, there is a possibility that a part of the invisible aligner corresponding to teeth that have not erupted is covered by the invisible aligner. As the teeth erupt, a corresponding position of the invisible aligner may not have enough space for eruption of the teeth due to covering adjacent gums, which may affect normal eruption of the teeth or make the invisible aligner unable to be worn after the teeth erupt.

Some methods in the prior art adopt a follow-up design for eruption space and tooth eruption, but there are certain problems with the above methods. For example, there are many comprehensive factors in a process of tooth eruption in the patient's mouth. If the eruption space is not designed reasonably in design of an orthodontic plan, there is a risk that the eruption space and the teeth may come into contact with each other and generate forces that affect the normal eruption of the teeth. In addition, if the prediction for tooth eruption speed and eruption parameters is not accurate during the follow-up design process, there may be errors in the eruption design, resulting in the patient being unable to wear the appliance normally.

All of these effects are undesirable during the orthodontic treatment process. Therefore, it is of great significance to develop an orthodontic system that has a simple eruption space design and does not affect normal growth and eruption of the teeth as the orthodontic treatment plan proceeds, as well as a design method thereof.

US20180360567A1 discloses a relevant technology regarding to systems and methods for detecting the eruption state of a target tooth. A patient's dentition may be scanned and/or segmented. A target tooth may be identified. Dental features, principal component analysis (PCA) features, and/or other features may be extracted and compared to those of other teeth, such as those obtained through automated machine learning systems. A detector can identify and/or output the eruption state of the target tooth, such as whether the target tooth is a fully erupted primary tooth, a permanent partially erupted/un-erupted tooth, or a fully erupted permanent tooth

US20160135924A1 discloses a relevant technology regarding to a method which includes to receive, via a computing device, data representing a plurality of teeth, identify data indicating which of the plurality of teeth are unerupted or erupting, predict at least one characteristic of a tooth of the unerupted or erupting teeth after they have fully erupted using one or more tooth eruption prediction factors, generate new data repre-senting the unerupted or erupting teeth in multiple states of eruption based upon the predicted at least one characteristic of the fully erupted teeth, and generate a series of incremental tooth arrangements with the new data to define a proposed orthodontic treatment based on the new data representing the unerupted or erupting teeth in multiple states of eruption.

### SUMMARY

The present application aims to provide an orthodontic system and a design method thereof, which can be applied to orthodontic treatment of adolescents during a period of tooth replacement to allow simultaneous tooth eruption during correction of dental malformations by a shell-like dental appliance. The invention is set out in the appended set of claims.

An orthodontic system is provided according to the present application, the orthodontic system includes at least one shell-like dental appliance configured to gradually adjust each of teeth other than unerupted teeth from an initial position to a target orthodontic position according to an orthodontic plan while allowing the teeth to erupt naturally. Each of the at least one shell-like dental appliance includes a dental appliance body including a geometric structure for accommodating multiple maxillary teeth or multiple mandibular teeth, and the dental appliance body is further provided with at least one eruption portion for accommodating one or more teeth not grown to preset eruption parameters; as the orthodontic plan proceeds, the eruption portion on each of the at least one shell-like dental appliances has a constant column structure, and there is a gap between an inner surface of each of the at least one eruption portion and an outer surface of each of the one or more teeth not grown to preset eruption parameters; where the preset eruption parameters include parameters of a tooth or teeth formed after full eruption of one or more ungrown or incompletely grown teeth.

According to the invention, the preset eruption parameters include a size, a position, a shape and an orientation of the tooth or teeth formed after the full eruption of the one or more ungrown or incompletely grown teeth.

The constant column structure is arranged based on a size, a position, a shape and an orientation of a tooth or teeth formed after full eruption of the one or more teeth not grown to the preset eruption parameters.

Specifically, as the orthodontic plan proceeds, adjacent teeth of the one or more teeth not grown to the preset eruption parameters is subjected to orthodontic movement, and thus there is an appropriate adjustment for the column structure of the eruption portion for a smooth transition to allow a smooth connection between the eruption portion and other parts of the shell-like body. In some embodiments, the column structure has a size 1.02 to 1.05 times of a size of the tooth or teeth formed after the full eruption of the one or more teeth not grown to the preset eruption parameters; the column structure is oriented at an angle of 0 to 5 degrees to a long axis of the tooth or teeth formed after the full eruption of the one or more teeth not grown to the preset eruption parameters; the column structure is positioned at an offset of 0 to 1 mm from a coordinate value in a spatial three-dimensional coordinate system of each of vertices that constitute a position of the tooth or teeth formed after the full eruption of the one or more teeth not grown to the preset eruption parameters; the column structure is shaped at an offset of 0 to 1 mm from a coordinate value in the spatial three-dimensional coordinate system of each of vertices that constitute a shape of the tooth or teeth formed after the full eruption of the one or more teeth not grown to the preset eruption parameters. The constant or substantially constant column structure as described in this embodiment is achieved in the above range of size, position, shape, and orientation.

In some embodiments, the constant column structure is further arranged based on a size, a position, a shape and an orientation of a tooth or teeth on an opposite jaw corresponding to the one or more teeth not grown to the preset eruption parameters, so that the constant or substantially constant column structure does not interfere with a maxillomandibular occlusal relationship.

In some embodiments, the constant column structure is arranged based on both first preset parameters of mesial adjacent teeth and second preset parameters of distal adjacent teeth of the one or more teeth not grown to the preset eruption parameters.

In some specific embodiments, the first preset parameters include the following parameters of the mesial adjacent teeth: a maximum dimension in a buccolingual diameter direction, a maximum dimension in a mesial-distal direction, and a maximum dimension of a height of a tooth in a long axis direction; and the second preset parameters include the following parameters of the distal adjacent teeth: a maximum dimension in the buccolingual diameter direction, a maximum dimension in the mesial-distal direction, and a maximum dimension of the height of the tooth in the long axis direction.

In some embodiments, the constant column structure includes a labial/buccal surface, a lingual surface and an occlusal surface, where the labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth.

In some embodiments, the constant column structure is further arranged based on a size, a position, a shape, and an orientation of teeth on an opposite jaw corresponding to the one or more teeth not grown to the preset eruption parameters, so that the constant or substantially constant column structure does not interfere with the maxillomandibular occlusal relationship.

In some embodiments, the constant column structure is arranged based on third preset parameters of distal adjacent teeth and mesial adjacent teeth of the one or more teeth not grown to the preset eruption parameters.

In some specific embodiments, the third preset parameters include: a maximum dimension in a buccolingual diameter direction, a maximum dimension in a medial-distal direction of the distal adjacent teeth, and a maximum dimension of a height of a tooth in a long axis direction of the mesial adjacent teeth.

In some embodiments, the constant column structure includes a labial/buccal surface, a lingual surface and an occlusal surface, where the labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth.

In some embodiments, the constant column structure is further arranged based on a size, a position, a shape, and an orientation of teeth on an opposite jaw corresponding to the one or more teeth not grown to the preset eruption parameters, so that the constant or substantially constant column structure does not interfere with the maxillomandibular occlusal relationship.

In some embodiments, the constant column structure is a cylindrical structure, an elliptical cylindrical structure, or a multi-prismatic column structure with not less than four lateral ribs
In some embodiments, a geometric structure on the shell-like dental appliance is configured to gradually adjust each of the teeth other than the unerupted teeth from the initial position to the target orthodontic position.

A design method of an orthodontic system is further provided according to the present application, including following operations S1 to S4.
S1. obtaining a digital dental model: obtaining a digital dental model, where the digital dental model includes a digital tooth model and a digital gingival model;
S2. segmenting and identifying the digital dental model: segmenting the digital dental model into a separate digital gingival model and a single digital crown model; identifying and marking data indicating teeth which do not erupt or do not fully erupt;
S3. virtually designing an orthodontic plan: designing the single digital crown model virtually so that the single digital crown model gradually changes from an initial position to a target orthodontic position to obtain a series of intermediate digital dental models;
S4. designing the orthodontic system:
   designing at least one shell-like dental appliance configured to gradually adjust each of teeth other than unerupted teeth from an initial position to a target orthodontic position according to an orthodontic plan while allowing the teeth to erupt naturally. Each of the at least one shell-like dental appliance includes a dental appliance body including a geometric structure for accommodating multiple maxillary teeth or multiple mandibular teeth, and the dental appliance body is further provided with at least one eruption portion for accommodating one or more teeth not grown to preset eruption parameters;
   as the orthodontic plan proceeds, the eruption portion on each of the at least one shell-like dental appliances has a constant or substantially constant column structure, and there is a gap between an inner surface of each of the at least one eruption portion and an outer surface of each of the one or more teeth not grown to preset eruption parameters; where the preset eruption parameters include parameters of a tooth or teeth formed after full eruption of one or more ungrown or incompletely grown teeth.

In one embodiment of the above design method, the geometric structure on the at least one shell-like dental appliance other than the eruption portion is configured to gradually adjust each of the teeth other than the unerupted teeth from the initial position to the target orthodontic position.

In addition, in a method for preparing an orthodontic system **a** shell-like dental appliance in the orthodontic system obtained according to the design method described above is prepared by using a thermocompression film molding or an additive preparing process to obtain a series of the shell-like dental appliances.

The present application has the following advantages over the prior art.

The orthodontic system for invisible orthodontics provided according to the present application includes a shell-like dental appliance. The shell-like dental appliance includes a dental appliance body, and the dental appliance body is further provided with an eruption portion, and the eruption portion on each of the shell-like dental appliances has a constant or substantially constant column structure as the orthodontic plan proceeds. The shell-like dental appliance has the effect of correcting dental malformations, and at the same time, the eruption portion of the dental appliance body is provided to receive the teeth not grown to the preset eruption parameters. The gap between the eruption portion and the teeth not grown to the preset eruption parameters is provided so that the dental appliance body, when worn, reserves space for the teeth to grow over the one or more teeth not grown to the preset eruption parameters. Therefore, each of the shell-like dental appliances of the entire orthodontic system does not interfere with the natural growth of the teeth when worn. In addition, the present application provides the eruption portion with the constant or substantially constant column structure, which makes design and use of each dental appliance and the eruption portion simpler. The eruption portion can be used as a standard attachment, which can be inserted on the dental model by selecting the standard attachment during use.

Implementation of any aspect of the present application can achieve some or all the above advantages.

### BRIEF DESCRIPTION OF THE DRAWINGS

FIG. 1 is schematic structural view of a shell-like dental appliance in a front view according to a first embodiment of the present application;
FIG. 2 is a schematic structural view of a shell-like dental appliance in a side view according to the first embodiment of the present application;
FIG. 3 is a schematic structural view of another shell-like dental appliance in a front view according to the first embodiment of the present application;
FIG. 4 is a schematic structural view of another shell-like dental appliance in a side view according to the first embodiment of the present application;
FIG. 5 is a schematic structural view of a shell-like dental appliance according to the first embodiment of the present application when being worn.
FIG. 6 is a schematic diagram of a design method of an orthodontic system according to a second embodiment of the present application;
FIG. 7 is a schematic structural view of a shell-like dental appliance in a front view according to the second embodiment of the present application;
FIG. 8 is a schematic structural view of a shell-like dental appliance in a side view according to the second embodiment of the present application;
FIG. 9 is a schematic structural view of another shell-like dental appliance in a front view according to the second embodiment of the present application;
FIG. 10 is a schematic structural view of another shell-like dental appliance in a side view according to the second embodiment of the present application;
FIG. 11 is a schematic structural view of a shell-like dental appliance in a front view according to a fourth embodiment of the present application;
FIG. 12 is a schematic structural view of a shell-like dental appliance in a side view according to the fourth embodiment of the present application;
FIG. 13 is a schematic structural view of a shell-like dental appliance in a front view according to a fifth embodiment of the present application;
FIG. 14 is a schematic structural view of a shell-like dental appliance in a side view according to the fifth embodiment of the present application.

Reference numerals: shell-like dental appliance (100, 200, 400, 500); eruption portion (120, 220), dental appliance body (110, 210, 410, 510); eruption cavity (420, 520).

### DETAILED DESCRIPTION OF THE EMBODIMENTS

In the description of the present application, it should be noted that "one or more teeth not grown to preset eruption parameters", i.e., "one or more ungrown or incompletely grown teeth", is also referred to as "erupting tooth" or "erupted tooth".

In the description of the present application, it should be noted that orientation or positional relationships indicated by the terms "center", "up", "down", "left", "right", "vertical", "horizontal", "inside", "outside", and the like are based on orientation or positional relationships shown in the accompanying drawings, which is only intended to facilitate and simplify the description of the present application, rather than indicating or implying that the device or element referred to must have a particular orientation, be constructed and operated in a particular orientation, and therefore should not be construed as a limitation on the present application. In addition, the terms "first", "second", and "third" are used for descriptive purposes only and should not be construed as indicating or implying relative importance.

As used in this specification, the singular forms "one", "a", and "the" include plural objects, unless expressly stated otherwise.

The present application is further described below in combination with specific embodiments.

### First embodiment

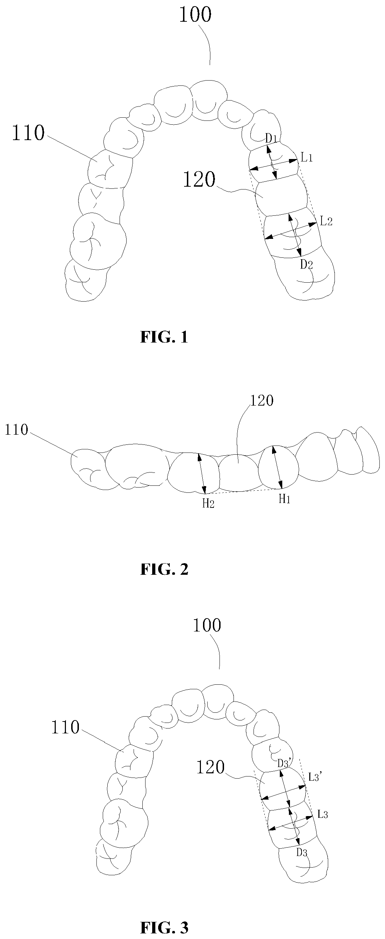

This embodiment provides an orthodontic system including at least one shell-like dental appliance 100 configured to gradually adjust each of teeth other than unerupted teeth from an initial position to a target orthodontic position according to an orthodontic plan while allowing the teeth to erupt naturally according to an orthodontic plan. Referring to FIG. 1 to FIG. 5, which are schematic structural views of the shell-like dental appliance of this embodiment, the shell-like dental appliance includes a dental appliance body 110. The dental appliance body 110 includes a geometric structure for accommodating multiple maxillary teeth or multiple mandibular teeth. The dental appliance body 110 is further provided with at least one eruption portion 120 for accommodating one or more teeth not grown to preset eruption parameters. The eruption portion 120 on each of the shell-like dental appliances 100 has a constant or substantially constant column structure as the orthodontic plan proceeds, and a gap is set between an inner surface of the eruption 120 and an outer surface of the teeth not grown to the preset eruption parameters. The preset eruption parameters include parameters of a tooth or teeth formed after full eruption of one or more ungrown or incompletely grown teeth.

The orthodontic system for invisible orthodontics provided in this embodiment is suitable for orthodontic treatment of adolescents in a period of tooth replacement. Due to a long period of an orthodontic plan, six months or even longer, patients in the period of tooth replacement need to design a space used to accommodate erupting teeth, influence of the erupting teeth on the orthodontic plan should be considered. When designing the orthodontic system, it is ensured that the erupting teeth are not subjected to forces generated by interaction with the shell-like dental appliance, which affects the eruption. Otherwise, the shell-like body for orthodontic treatment may cover gingiva above the erupting teeth and would inhibit the growth of the erupting teeth.

Specifically, the shell-like dental appliance 100 (also noted as a dental appliance) of this embodiment has an effect of correcting tooth malocclusion while the eruption portion 120 on the dental appliance body 110 is provided to receive the teeth not grown to the preset eruption parameters. A gap between the inner surface of the eruption portion 120 and the teeth not grown to the preset eruption parameters allows the dental appliance body 110 to reserve a space for tooth growth above the one or more teeth not grown to the preset eruption parameters when worn. Therefore, the shell-like dental appliance 100 does not interfere with the natural growth of the teeth when worn. Herein, the orthodontic system of this embodiment is suitable for an orthodontic plan with multiple orthodontic stages, where the eruption portion 120 of each of the dental appliance body 110 has a constant or substantially constant column structure as the orthodontic plan proceeds, so that each of the dental appliance bodies 110 does not touch the teeth never grown to the preset eruption parameters throughout the orthodontic system. In addition, the structure of the eruption portion 120 makes design and use of the respective dental appliance and the eruption portion 120 simpler, and it (eruption portion 120) is used as a standard attachment that can be inserted on a dental model by selecting that standard attachment when in use.

In some embodiments, the preset eruption parameters include a size, a position, a shape, and an orientation of the tooth or teeth formed after the full eruption of the one or more ungrown or incompletely grown teeth. Herein, the size, position, shape, and orientation may be a size, position, shape, and orientation of the incompletely grown teeth obtained based on CBCT of a patient, or a size, position, shape, and orientation of the ungrown or incompletely grown teeth obtained based on one or more denture banks, or a size, position, shape, and orientation of the ungrown or incompletely grown teeth based on statistics under big data.

In some embodiments, the constant or substantially constant column structure is arranged based on the size, position, shape, and orientation of the tooth or teeth formed after the full eruption of the one or more teeth not grown to the preset eruption parameters.

Specifically, as the orthodontic plan proceeds, adjacent teeth of the one or more teeth not grown to the preset eruption parameters are subjected to orthodontic movement, and thus there is an appropriate adjustment of the column structure of the eruption portion 120 for a smooth transition to allow a smooth connection between the eruption portion 120 and other parts of the shell-like body.

In some embodiments, the column structure has a size 1.02 to 1.05 times of a size of the tooth or teeth formed after the full eruption of the one or more teeth not grown to the preset eruption parameters. More specifically, the size of the column structure is based on the size of the tooth after full eruption, which is a fixed size and does not change. Therefore, the column structure designed based on the fixed size and further enlarged by 1.02 to 1.05 times has a larger size than that of the tooth or teeth formed after the full eruption of the one or more teeth not grown to the preset eruption parameters, ensuring that the one or more teeth not grown to the preset eruption parameters always remain out of contact with the inner surface of the eruption portion 120 (eruption cavity) during the eruption process.

The column structure is oriented at an angle of 0 to 5 degrees to a long axis of the tooth or teeth formed after the full eruption of the one or more teeth not grown to the preset eruption parameters. More specifically, the orientation of the column structure is based on an orientation of the tooth or teeth formed after the full eruption of the one or more teeth not grown to the preset eruption parameters, which is a definite orientation. Therefore, the orientation of the column structure based on this definite orientation is designed to have a greater range of angles than the orientation of the tooth or teeth formed after the full eruption of the one or more teeth not grown to the preset eruption parameters. That is, the angular orientation is expanded by 0 to 5 degrees based on a long axis of unerupted or incompletely erupted teeth, ensuring that the unerupted or incompletely erupted teeth remain out of contact with the eruption portion 120 (eruption cavity) formed during the eruption process.

The column structure is positioned at an offset of 0 to 1 mm from a coordinate value in a spatial three-dimensional coordinate system of each of vertices that constitute a position of the tooth or teeth formed after the full eruption of the one or more teeth not grown to the preset eruption parameters. More specifically, based on the position after full eruption of the erupting tooth, which is a definite position, the position of each of the vertices of the column structure is offset outwardly from an inside of the eruption portion 120. It should be noted that the design of the orthodontic system is carried out based on a digital dental model which is composed of multiple triangular facets in a unified three-dimensional coordinate system, and each vertex in each of the triangular facets has its corresponding spatial coordinate value in the three-dimensional coordinate system. The position of the tooth or teeth formed after full eruption of the one or more teeth not grown to the preset eruption parameters is determined based on the spatial coordinate values of each vertex that constitute the position. That is, the individual vertex of the erupting tooth is used as a reference for an expansion of the 0 to 1 mm offset, which ensures that the erupting teeth remain out of contact with the eruption portion 120 (erupted cavity) formed during the eruption process.

The column structure is shaped at an offset of 0 to 1 mm from a coordinate value in the spatial three-dimensional coordinate system of each of vertices that constitute a shape of the tooth or teeth formed after the full eruption of the one or more teeth not grown to the preset eruption parameters. More specifically, the shape of the column structure is based on the shape of the tooth or teeth formed after the full eruption of the one or more teeth not grown to the preset eruption parameters, which is a definite shape, and therefore the shape of the column structure is determined based on the above-mentioned definite shape. It should be noted that the design of the orthodontic system is carried out based on the digital dental model, which is composed of multiple triangular facets in the uniform three-dimensional coordinate system, and each vertex in each of the triangular facets has its corresponding spatial coordinate value in the three-dimensional coordinate system. The shape of the tooth or teeth formed after the full eruption of the one or more teeth not grown to the preset eruption parameters is based on the spatial coordinate values of each vertex that constitute the shape, while the shape of the column structure has a larger range of offset than the shape of the tooth or teeth formed after the full eruption of the teeth not grown to the preset eruption parameters. That is, the individual vertex of the unerupted or incomplete erupting teeth is used as a reference for an expansion of 0 to 1 mm offset, which ensures that the eruption portion 120 (eruption cavity) has a smooth transition to its adjacent geometric structures and remains out of contact with the erupting tooth throughout the eruption process. The constant or substantially constant column structure as described in this embodiment is achieved with the range of size, position, shape, and orientation described above.

In some embodiments, the constant or substantially constant column structure is further arranged based on a size, a position, a shape, and an orientation of a tooth or teeth on an opposite jaw corresponding to the one or more teeth not grown to the preset eruption parameters, so that the constant or substantially constant column structure does not interfere with maxillomandibular occlusal relationship setting. Herein, the corresponding teeth on the opposite jaw refer to teeth on the opposite jaw that are occluded with the one or more teeth not grown to the preset eruption parameters, by which an occlusal surface of the constant or substantially constant column structure is designed. The occlusal surface of the constant or substantially constant column structure is designed as a flat, curved, or a structure with protrusions and recesses to be matched with opposing teeth. The occlusal surface of the eruption portion 120 is designed according to the opposing teeth, so that the eruption portion 120 is cuspid-fossa matching with the opposing teeth, or so that the occlusal surface of the eruption portion 120 is provided with protrusions and recesses to be matched with the occlusal surface of the opposing teeth.

In some embodiments, the constant or substantially constant column structure is arranged based on both first preset parameters of mesial adjacent teeth and second preset parameters of distal adjacent teeth of the one or more teeth not grown to the preset eruption parameters.

In some specific embodiments, the first preset parameters include the following parameters of the mesial adjacent teeth: a maximum dimension L₁ in a buccolingual diameter direction, a maximum dimension D₁ in a mesial-distal direction and a maximum dimension H₁ of a height of a tooth in a long axis direction; the second preset parameters include the following parameters of the distal adjacent teeth: a maximum dimension L₂ in the buccolingual diameter direction, a maximum dimension D₂ in the mesial-distal direction and a maximum dimension H₂ of the height of the tooth in the long axis direction. In one embodiment, as shown in FIG. 1 and FIG. 2, the eruption portion 120 of the shell-like dental appliance 100 is provided to encase eruption of a second premolar. At this time, the first preset parameters are the maximum dimension L₁ in the buccolingual diameter direction, the maximum dimension D₁ in the mesial-distal direction, and the maximum dimension H₁ in the height of the tooth in the long axis direction of a first premolar; the second preset parameters are the maximum dimension L₂ in the buccolingual diameter direction, the maximum dimension D₂ in the mesial-distal direction, and the maximum dimension H₁ of the height of the tooth in the long axis direction of a first molar. The determination of the constant or substantially constant column structure based on the first and second preset parameters is performed by determining the dimension of the constant or substantially constant column structure in the buccolingual diameter direction by means of arithmetic averaging, weighted averaging, etc. between L₁ and L₂; determining the dimension of the constant or substantially constant column structure in the mesial-distal direction by means of arithmetic averaging, weighted averaging, etc. between D₁ and D₂, determining the dimension of the constant or substantially constant column structure in the long axis direction of the tooth by means of arithmetic averaging, weighted averaging, etc. between H₁ and H₂. As a result, the eruption portion 120 may fit the size and shape of the one or more teeth not grown to the preset eruption parameters throughout the orthodontic plan to the maximum extent.

In some embodiments, the constant or substantially constant column structure includes a labial/buccal surface, a lingual surface, and an occlusal surface. Herein, the labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth. This setup structure allows the shell-like dental appliance 100 to have a smoother shell-like structure that wraps around the teeth and less foreign body sensation in the mouth when worn by the patient. It should be noted that the constant or substantially constant column structure means that the shape, size, position, and orientation of the eruption portion 120 are consistent. Since the teeth may move as the orthodontic treatment proceeds during the orthodontic process of wearing a series of shell-like dental appliances, the eruption portion 120 may have a partial spatial adaptation for a smooth transition connection with a geometric structure of the adjacent teeth. Alternatively, in some embodiments, the constant or substantially constant column structure may also include only the labial/buccal and lingual surfaces, but not include the occlusal surface.

In some embodiments, the constant or substantially constant column structure is further arranged based on the size, position, shape, and orientation of the teeth on the opposite jaw corresponding to the one or more teeth not grown to the preset eruption parameters so that the constant or substantially constant column structure does not interfere with the maxillomandibular occlusal relationship setting. The occlusal surface of the eruption portion 120 of this embodiment is designed as a flat, curved or a structure with protrusions and recesses to be matched with the opposing teeth. The occlusal surface of the eruption portion 120 is designed according to the opposing teeth so that the eruption portion 120 is cuspid-fossa matching with the opposing teeth, or so that the occlusal surface of the eruption portion 120 is a structure with protrusions and recesses to be matched with the occlusal surface of the opposing teeth.

In some embodiments, the constant or substantially constant column structure is arranged based on third preset parameters of distal adjacent teeth and mesial adjacent teeth of the one or more teeth not grown to the preset eruption parameters.

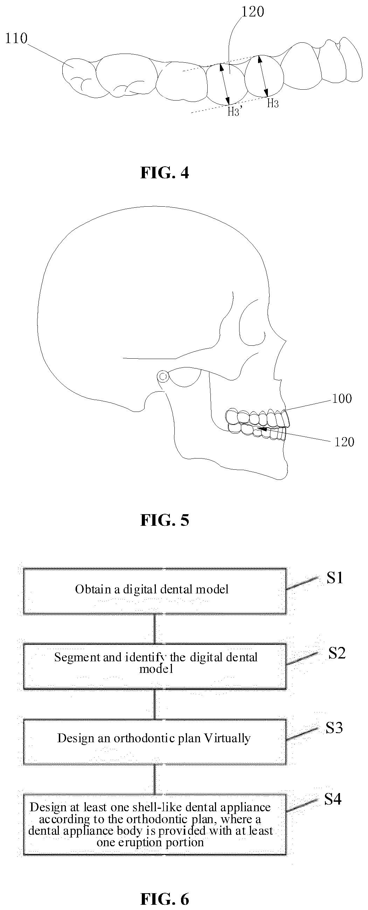

In some specific embodiments, the third preset parameters include: a maximum dimension in a buccolingual diameter direction, a maximum dimension in a mesial-distal direction of the distal adjacent teeth, and a maximum dimension of a height of a tooth in a long axis direction of the mesial adjacent teeth. In some specific embodiments, as shown in FIG. 3 and FIG. 4, the eruption portion 120 on the shell-like dental appliance is provided to encase the eruption of the second premolar. At this time, the third preset parameters are a maximum dimension L₃ in the buccolingual diameter direction, a maximum dimension D₃ in the mesial-distal direction, and a maximum dimension H₃ of the height of the tooth in the long axis direction of the first molar, which are used to determine a maximum dimension L₃' in the buccolingual diameter direction, a maximum dimension D₃' in the mesial-distal direction, and a maximum dimension H₃' in the height of the long-axis direction of the mesial adjacent teeth of the eruption portion 120 of the second premolar. The column structure designed based on the maximum dimension in the buccolingual diameter direction, the maximum dimension in the medial-distal direction of the distal adj acent teeth and the maximum dimension of the height of the teeth in the long-axis direction of the medial adjacent tooth is sufficient to accommodate the one or more teeth not grown to the preset eruption parameters, and such that the dimension of the column structure is slightly larger than that of the one or more teeth not grown to the preset eruption parameters, so as to ensure that a gap can be set between the column structure and the erupting teeth.

In some embodiments, the constant or substantially constant column structure includes a labial/buccal surface, a lingual surface, and an occlusal surface. The labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth. This setup structure allows the shell-like dental appliance 100 to have a smoother shell-like structure that wraps around the teeth and less foreign body sensation in the mouth when worn by the patient.

In some embodiments, the constant or substantially constant column structure is further arranged based on the size, position, shape, and orientation of the teeth on the opposite jaw corresponding to the one or more teeth not grown to the preset eruption parameters so that the constant or substantially constant column structure does not interfere with the maxillomandibular occlusal relationship setting. The occlusal surface of the eruption portion 120 of this embodiment is designed as a flat, curved or a structure with protrusions and recesses to be matched with the opposing teeth. The occlusal surface of the eruption portion 120 is designed according to the opposing teeth so that the eruption portion 120 is cuspid-fossa matched with the opposing teeth, or so that the occlusal surface of the eruption portion 120 is a structure with protrusions and recesses to be matched with the occlusal surface of the opposing teeth.

In some embodiments, the constant or substantially constant column structure is a cylindrical structure, an elliptical cylinder, or a multi-pronged column structure with not less than four lateral ribs, which may be set according to a number and type of erupting teeth, or can be adaptively selected according to an eruptive gap existing between adjacent teeth.

In some embodiments, a geometric structure on the shell-like dental appliance 100 other than the eruption portion 120 causes teeth other than unerupted teeth to be gradually adjusted from the initial position to the target orthodontic position. That is, the eruption portion 120 in this embodiment only reserves space for growth of the one or more teeth not grown to the preset eruption parameters, so that the shell-like dental appliance 100 does not interfere with the natural growth of the erupting teeth, but the eruption portion 120 in this embodiment does not have a corrective effect on abnormally growing erupting teeth. That is, if the one or more teeth not grown to the preset eruption parameters are abnormally growing teeth, the eruption portion 120 of the present embodiment is also set according to the erupting teeth without corrective intervention. Instead, the geometric structure on the shell-like dental appliance 100 other than the eruption portion 120 is configured to gradually adjust the teeth other than the unerupted teeth from the initial position to the target orthodontic position. That is, the geometric structure other than the eruption portion 120 has a corrective effect on remaining teeth other than the erupting teeth, allowing the teeth to be aligned without interfering with the eruption of the teeth.

### Second embodiment

This embodiment provides a design method of an orthodontic system. As shown in FIG. 6, which is a schematic diagram of the design method of this embodiment, the orthodontic system is any of the orthodontic systems as described in Embodiment 1, and the design method includes operations S1 to S4 as follows.

In operation S1, a digital dental model is obtained, and the digital dental model includes a digital tooth model and a digital gingival model.

In operation S2, the digital dental model is segmented into a separate digital gingival model and multiple digital crown models, and data indicating unerupted or incompletely erupted teeth is identified and marked.

In operation S3, the single digital crown model is designed virtually so that the single digital crown model gradually changes from an initial position to a target orthodontic position to obtain a series of intermediate digital dental models.

In operation S4, at least one shell-like dental appliance 200 configured to gradually adjust teeth from the initial position to the target orthodontic position while allowing the teeth to erupt is designed according to the orthodontic plan where the shell-like dental appliance 200 includes a dental appliance body 210, the dental appliance body 210 includes a geometric structure for accommodating multiple maxillary teeth or multiple mandibular teeth, the dental appliance body 210 is further provided with at least one eruption portion 220 for accommodating one or more teeth not grown to preset eruption parameters. As the orthodontic plan proceeds, the eruption portion 220 on each of the shell-like dental appliances 200 has a constant or substantially constant column structure, and a gap is set between an inner surface of the eruption portion 220 and an outer surface of the teeth not grown to the preset eruption parameters.

Specifically, in the design method described in this embodiment, a digital maxillary dental model and a mandibular dental model in operation S1 is obtained by any of the following methods: a laminar X-ray scanning (CAT scanning), a digital tomography (CT) scanning, a cone beam CT scanning (CBCT), a magnetic resonance imaging (MRI), an intraoral optical scanning, etc. to obtain a digital model representing an original tooth layout. Alternatively, a plaster cast of the patient's tooth is made by conventional means, and then the plaster cast may be scanned by a scanning device such as a laser scanning device, a CT scanning device to obtain the digital model representing the original tooth layout.

Specifically, in the design method described in this embodiment, the digital dental model in operation S2 is segmented in the following non-limiting embodiments.

In S200, a first type of feature points on the digital dental model to be segmented are selected, and the digital dental model is a triangular facet model.

In S201, a second type of feature points in the digital dental model are classified based on the first type of feature points and a tooth to which each of the second type of feature points belongs is determined.

In S202, the second type of feature points belonging to each tooth are merged separately to obtain a digital tooth region of each single tooth after segmentation of the digital dental model.

The above-mentioned first type of feature points are triangular facet vertices selected based on the digital dental model and used to guide segmentation of each individual tooth in a jaw, and the second type of feature points are triangular facet vertices selected based on the digital dental model and used to characterize an overall shape of the digital dental model. That is, the first type of feature points are used to guide the segmentation of the jaw, and the second type of feature points are feature points used in the specific segmentation of the jaw. Through the segmentation guidance of the first type of feature points, the second type of feature points can be accurately classified to each tooth, thereby improving segmentation accuracy of the jaw.

Segmentation for a single tooth is achieved by integrally selecting the first type of feature points on the digital dental model, and then classifying and reassembling the second type of feature points on the digital dental model based on the first type of feature points. The two types of feature points are integrally selected based on the digital dental model, and classification information of the feature points covers overall classification features of the digital dental model. Therefore, even if there is noisy data in the model, the noisy data will be evenly distributed to the global data, resulting in a high fault tolerance of the entire segmentation method. In addition, the single tooth can be segmented more accurately to ensure integrity of each tooth.

Further, in operation S2, the teeth on the tooth model after cutting are identified and marked, and the specific implementation of identifying and marking the data indicating the teeth not grown to the preset eruption parameters is as follows: firstly, a tooth position is identified, and then a volume of the identified tooth is compared with a volume of a standard tooth, and when the volume of the identified tooth is smaller than the volume of the corresponding standard tooth within a certain threshold, the tooth is marked as the tooth not grown to the preset eruption parameters. The threshold is, for example, half of the volume of the standard tooth.

More specifically, the tooth position identification may use the following methods. In operation 1, a first priori model, a second priori model and a third priori model are built. Herein, the first priori model includes collecting a spacing of each two adjacent teeth in the existing tooth model and the number of missing teeth corresponding to the spacing, and calculating probability distribution function values for the spacing of different numbers of missing teeth. The second priori model includes collecting feature vector representing a position of each tooth in the existing dental model, and calculating a probability distribution function value for the feature vector at least representing position of the teeth with the same number. The third prior model includes collecting tooth position arrangement of each two adjacent teeth in the existing dental model without missing teeth, or with different numbers of missing teeth, and calculating a probability distribution function value for the tooth position arrangement. In operation 2, a feature vector representing the position of each tooth of the tooth model to be tested and a spacing between two adjacent teeth are acquired. In operation 3, the tooth position of the tooth model to be tested is determined based on Hidden Markov Model. The identification of the tooth position is performed according to the above method, after which the tooth volume is compared with that in the standard tooth model according to the tooth position marker, such as using a change in a coordinate value of a feature point within a certain threshold, and determining whether the tooth is marked as the tooth not grown to the preset eruption parameters.

Specifically, in the design method described in this embodiment, in operation S3, the single digital crown model is virtually designed so that the single digital crown model is gradually changed from an initial position to a target orthodontic position to obtain a series of intermediate digital dental models. Herein, the initial position is an original layout of the teeth before an orthodontic treatment begins, or any stage of the orthodontic treatment process, and the target orthodontic position is any stage after the orthodontic treatment begins, which is a later stage or later stages of the original layout of the teeth. The target orthodontic position is a position of the final orthodontic effect obtained by the physician and medical designer according to the patient's request and intraoral situation, or the target orthodontic position is recommended according to an intraoral digital design software, based on similar cases, or more targeted adjustments is made to patient treatment according to recommendation results.

Specifically, in the design method described in this embodiment, in operation S4, the orthodontic system is designed, in which at least one shell-like dental appliance 200 that gradually adjusts the teeth from the initial position to the target orthodontic position while allowing the teeth to erupt according to the orthodontic plan is designed. The shell-like dental appliance 200 is used in any orthodontic phase of the orthodontic plan, such as an initial phase or a final phase of the orthodontic treatment. The eruption portion 220 having the constant or substantially constant column structure is provided as a standard attachment for easy use by clinicians or other users who may directly select this standard attachment for insertion into the dental model when designing the shell-like dental appliance 200.

In some embodiments, the constant or substantially constant column structure is arranged based on a size, a position, a shape, and an orientation of the tooth or teeth formed after full eruption of the one or more teeth not grown to the preset eruption parameters.

Specifically, as the orthodontic plan proceeds, adjacent teeth of the one or more teeth not grown to the preset eruption parameters is subjected to orthodontic movement, and thus there is an appropriate adjustment of the column structure of the eruption portion 220 for a smooth transition to allow a smooth connection between the eruption portion 220 and other parts of the shell-like body. In some embodiments, a size of the column structure is 1.02 to 1.05 times of a size of the tooth or teeth formed after the full eruption of the one or more teeth not grown to the preset eruption parameters. More specifically, the size of the column structure is based on the size of the tooth or teeth formed after the full eruption of the one or more teeth not grown to the preset eruption parameters, which is a fixed size and does not change. Therefore, the column structure designed based on the fixed size mentioned above has a larger size than that of the tooth or teeth formed after the full eruption of the tooth or teeth not grown to the preset parameters, which ensures that the unerupted or incompletely erupted teeth remain out of contact with an eruption cavity formed during an eruption process.

The column structure is oriented at an angle of 0 to 5 degrees to an orientation of a long axis of the tooth or teeth formed after the full eruption of the one or more teeth not grown to the preset eruption parameters. More specifically, the orientation of the column structure is based on the orientation of the tooth or teeth formed after the full eruption of the one or more teeth not grown to the preset eruption parameters, which is a definite orientation. Therefore, the orientation of the column structure determined based on the definite orientation has a larger range of orientation angles compared with the orientation of the tooth or teeth formed after the full eruption of the teeth not grown to the preset eruption parameters. That is, the orientation angle is expanded by 0 to 5 degrees based on the long axis of the teeth not grown to the preset eruption parameters, ensuring that the unerupted or incompletely erupted teeth remain out of contact with the eruption portion 220 (eruption cavity) formed during the eruption process.

The column structure is positioned at an offset of 0 to 1 mm from a coordinate value in a spatial three-dimensional coordinate system of each of vertexes that constitute a position of the tooth or teeth formed after the full eruption of the one or more teeth not grown to the preset eruption parameters. More specifically, based on the position of the tooth or teeth formed after the full eruption of the one or more teeth not grown to the preset eruption parameters, which is a definite position, the position of each vertex of the column structure is offset outwardly from an inside of the eruption portion 120 based on the definite position. It should be noted that the design of the orthodontic system is carried out based on a digital dental model, which is composed of multiple triangular facets in a unified three-dimensional coordinate system, and each vertex in each of the triangular facets has its corresponding spatial coordinate value in the three-dimensional coordinate system. The position of the tooth or teeth formed after the full eruption of the one or more teeth not grown to the preset eruption parameters is determined based on the spatial coordinate values of each of the vertices that constitutes the position. That is, each vertex of the erupted tooth is used as a reference for the expansion of the 0 to 1 mm offset, which ensures that the erupting teeth remain out of contact with the eruption portion 120 (eruption cavity) formed during the eruption process.

The column structure is shaped at an offset of 0 to 1 mm from a coordinate value in the spatial three-dimensional coordinate system of each of vertexes that constitute a shape of the tooth or teeth formed after the full eruption of the one or more teeth not grown to the preset eruption parameters. More specifically, the shape of the column structure is based on the shape of the teeth formed after the full eruption of the teeth not grown to the preset eruption parameters, which is a definite shape. Therefore, the shape of the column structure is determined based on the above definite shape. It should be noted that the design of the orthodontic system is based on the digital dental model, which is composed of multiple triangular facets in the unified three-dimensional coordinate system, and each vertex in each of the triangular facets has its corresponding spatial coordinate value in the three-dimensional coordinate system. The shape of the tooth or teeth formed after the full eruption of the one or more teeth not grown to the preset eruption parameters is based on the spatial coordinate value of each of the vertices that constitute the shape. The shape of the column structure has a greater range of offset than the shape of the teeth formed after the full eruption of the teeth not grown to the preset eruption parameters. That is, each vertex of the unerupted or incompletely erupted teeth is used as a reference for the expansion of the 0 to 1 mm offset, so that the eruption portion 120 (eruption cavity) is smoothly transitioned to its adjacent geometric structures and remains out of contact with the erupting teeth during the eruption process. The constant or substantially constant column structure as described in this embodiment is achieved in the range of the size, position, shape, and orientation as described above.

In some embodiments, the constant or substantially constant column structure is further arranged based on a size, a position, a shape, and an orientation of teeth on an opposite jaw corresponding to the one or more teeth not grown to the preset eruption parameters, so that the constant or substantially constant column structure does not interfere with the maxillomandibular occlusal relationship setting. Herein, the corresponding teeth on the opposite jaw refer to teeth on the opposite jaw that are occluded with the one or more teeth not grown to the preset eruption parameters. The occlusal surface of the constant or substantially constant column structure is designed based on the corresponding teeth on the opposite jaw. The occlusal surface of the constant or substantially constant column structure is designed as a flat, curved, or a structure with protrusions and recesses to be matched with the opposing teeth. The occlusal surface of the eruption portion 220 is designed based on the opposing teeth so that the eruption portion 220 is cuspid-fossa matching with the opposing teeth, or so that the occlusal surface of the eruption portion 220 is a structure with protrusions and recesses to be matched with the occlusal surface of the opposing teeth.

In some embodiments, the constant or substantially constant column structure is arranged based on both first preset parameters of mesial adjacent teeth and second preset parameters of distal adjacent teeth of the one or more teeth not grown to the preset eruption parameters.

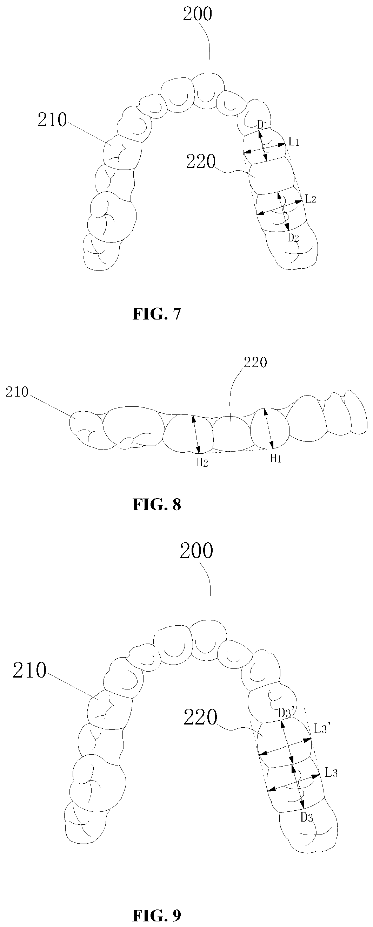

In some specific embodiments, the first preset parameters include the following parameters of the mesial adjacent teeth: a maximum dimension L₁ in a buccolingual diameter direction, a maximum dimension D₁ in a mesial-distal direction and a maximum dimension H₁ of a height of the tooth in a long axis direction. The second preset parameters include the following parameters of the distal adj acent teeth: a maximum dimension L₂ in the buccolingual diameter direction, a maximum dimension D₂ in the mesial-distal direction and a maximum dimension H₂ of the height of the tooth in the long axis direction. In one embodiment, as shown in FIG. 7 and FIG. 8, the eruption portion 220 of the shell-like dental appliance 200 is provided to encase eruption of a second premolar. At this time, the first preset parameters are a maximum dimension in the buccolingual diameter direction, a maximum dimension in the mesial-distal direction, and a maximum dimension of the height of the tooth in the long axis direction of a first premolar; the second preset parameters are a maximum dimension in the buccolingual diameter direction, a maximum dimension in the mesial-distal direction, and a maximum dimension of the height of the tooth in the long axis direction of the first premolar. The determination of the constant or substantially constant column structure based on the first preset parameters and the second preset parameters is performed by determining the dimension of the constant or substantially constant column structure in the buccolingual diameter direction by means of arithmetic averaging, weighted averaging, etc. between L₁ and L₂; determining the dimension of the constant or substantially constant column structure in the mesial-distal direction by means of arithmetic averaging, weighted averaging, etc. between D₁ and D₂; determining the dimensions of the constant or essentially constant column structure in the long axis of the teeth by means of arithmetic averaging, weighted averaging, etc. between H₁ and H₂. As a result, the eruption portion 220 may fit the size and shape of the one or more teeth not grown to the preset eruption parameters throughout the orthodontic plan to the maximum extent.

In some embodiments, the constant or substantially constant column structure includes a labial/buccal surface, a lingual surface, and an occlusal surface. The labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth. This setup structure allows the shell-like dental appliance 200 to have a smoother shell-like structure that wraps around the teeth and less foreign body sensation in the mouth when worn by the patient. It should be noted that the constant or substantially constant column structure means that the body shape, size, position, and orientation of the eruption portion 220 are consistent. Since the teeth may move as the orthodontic treatment proceeds during the orthodontic process of wearing a series of shell-like dental appliances, the eruption portion 120 may have a partial spatial adaptation for a smooth transition connection with a cavity of the adjacent teeth. In some embodiments, the constant or substantially constant column structure may only include the labial/buccal and lingual surfaces, but not include the occlusal surface.

In some embodiments, the constant or substantially constant column structure is further arranged based on the size, position, shape, and orientation of the teeth on the opposite jaw corresponding to the one or more teeth not grown to the preset eruption parameters so that the constant or substantially constant column structure does not interfere with the maxillomandibular occlusal relationship setting. The occlusal surface of the eruption portion 220 of this embodiment is designed as a flat, curved or a structure with protrusions and recesses to be matched with the opposing teeth. The occlusal surface of the eruption portion 220 is designed according to the opposing teeth so that the eruption portion 220 is cuspid-fossa matching with the opposing teeth, or so that the occlusal surface of this eruption portion 220 is a structure with protrusions and recesses to be matched with the occlusal surface of the opposing teeth.

In some embodiments, the constant or substantially constant column structure is arranged based on third preset parameters of distal adjacent teeth and mesial adjacent teeth of the one or more teeth not grown to the preset eruption parameters.

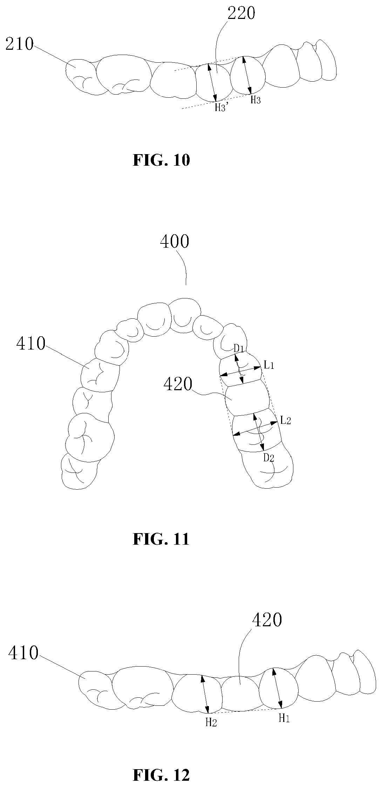

In some specific embodiments, the third preset parameters include: a maximum dimension in the buccolingual diameter direction, a maximum dimension in the mesial-distal direction of the distal adjacent teeth, and a maximum dimension of a height of a tooth in a long axis direction of the mesial adjacent teeth. In some specific embodiments, as shown in FIG. 9 and FIG. 10, the eruption portion 220 on the shell-like dental appliance is provided to encase the eruption of the second premolar. At this time, the third preset parameters are a maximum dimension L₃ in the buccolingual diameter direction, a maximum dimension D₃ in the medial-distal direction, and a maximum dimension H₃ of the height in the long-axis direction of the mesial adj acent teeth of the first molar, which may be used to determine a maximum dimension L₃' in the buccolingual diameter direction, a maximum dimension D₃' in the mesial-distal direction and a maximum dimension H₃' in the height of the long-axis direction of the mesial adjacent teeth of the eruption portion 220 of the second premolar. Thus, the column structure designed on the basis of the maximum dimension in the buccolingual diameter direction of the distal adjacent teeth, the maximum dimension in the medial-distal direction and the maximum dimension of the height in the long-axis direction of the medial adjacent teeth is sufficient to accommodate the one or more teeth not grown to the preset eruption parameters, and such that the dimension of the column structure designed is slightly larger than that of the one or more teeth not grown to the preset eruption parameters, so as to ensure that a gap may be set between the column structure and the erupting tooth.

In some embodiments, the constant or substantially constant column structure includes a labial/buccal surface, a lingual surface, and an occlusal surface. The labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal side of the mesial adjacent teeth and the distal adjacent teeth. This setup structure allows the shell-like dental appliance 200 to have a smoother shell-like structure that wraps around the teeth and less foreign body sensation in the mouth when worn by the patient.

In some embodiments, the constant or substantially constant column structure is further arranged based on the size, position, shape, and orientation of the teeth on the opposite jaw corresponding to the one or more teeth not grown to the preset eruption parameters so that the constant or substantially constant column structure does not interfere with the maxillomandibular occlusal relationship setting. The occlusal surface of the eruption portion 220 of this embodiment is designed as a flat, curved or a structure with protrusions and recesses to be matched with the opposing teeth. The occlusal surface of the eruption portion 220 may be designed according to the opposing teeth so that the eruption portion 220 is cuspid-fossa matching with the opposing teeth, or so that the occlusal surface of this eruption portion 220 is a structure with protrusions and recesses to be matched with the occlusal surface of the opposing teeth.

In some embodiments, the constant or substantially constant column structure is a cylindrical structure, an elliptical cylinder, or a multi-pronged column structure with not less than four lateral ribs, which may be set according to a number and type of erupting teeth, or may be adaptively selected according to an eruptive gap existing between adjacent teeth.

In some embodiments, a geometric structure on the shell-like dental appliance 200 other than the eruption portion 220 causes teeth other than unerupted teeth to be gradually adjusted from the initial position to the target orthodontic position. That is, the eruption portion 220 in this embodiment only reserves space for the growth of the one or more teeth not grown to the preset eruption parameters, so that the shell-like dental appliance 200 does not interfere with the natural growth of the erupting teeth, but the eruption portion 220 in this embodiment does not have a corrective effect on abnormally growing erupting teeth. That is, if the one or more teeth not grown to the preset eruption parameters are abnormally growing teeth, the eruption portion 220 of the present embodiment is also set according to the erupting teeth without corrective intervention. Instead, the geometric structure on the shell-like dental appliance 100 other than the eruption portion 120 causes the teeth other than the unerupted teeth to be gradually adjusted from the initial position to the target orthodontic position. That is, the geometric structure other than the eruption portion 120 has the corrective effect on the remaining teeth other than the erupted teeth, allowing the teeth to be aligned without interfering with the eruption of the teeth.

### Third embodiment

This embodiment also provides a method for preparing an orthodontic system in which a shell-like dental appliance in an orthodontic system obtained according to anyone of the design methods described in the second embodiment is prepared by using a thermocompression film molding or an additive preparing process to obtain a series of the shell-like dental appliances.

For example, when the thermos-compression film molding process is adopted, the specific preparing method includes: a 3D printing is carried out based on a digital dental model and a series of intermediate digital dental models to produce a solid dental model, after which the shell-like dental appliance containing a tooth shape is obtained by thermocompression film molding on the solid dental model, after which the shell-like dental appliance capable of accommodating teeth is subsequently obtained by cutting along or adjacent to a gingival line.

For example, when the additive preparing process is adopted, the specific preparing process is to print a digital model of the designed shell-like dental appliance using the 3D printing method.

### Fourth embodiment

This embodiment provides a method for predicting an eruption cavity of a shell-like dental appliance, in which at least one shell-like dental appliance 400 is designed to gradually adjust teeth from an initial position to a target orthodontic position while allowing the teeth to erupt according to an orthodontic plan. Herein, the shell-like dental appliance 400 includes a dental appliance body 410 with a geometric structure accommodating multiple maxillary teeth or multiple mandibular teeth and at least one eruption cavity 420 accommodating one or more teeth not grown to preset eruption parameters. The eruption cavity 420 is predicted based on both first preset parameters of mesial adj acent teeth and second preset parameters of distal adjacent teeth of the one or more teeth not grown to the preset eruption parameters, such that a gap is set between an inner surface of the eruption cavity 420 and an outer surface of the teeth not grown to the preset eruption parameters. The preset eruption parameters are designed to include parameters of a tooth or teeth formed after full eruption of one or more ungrown or incompletely grown teeth.

In some embodiments, the preset eruption parameters include a size, a position, a shape, and an orientation of the tooth or teeth formed after the full eruption of the one or more ungrown or incompletely grown teeth. Herein, the size, position, shape, and orientation may be a size, position, shape, and orientation of the incompletely grown teeth obtained based on CBCT of a patient, or a size, position, shape, and orientation of the ungrown or incompletely grown teeth obtained based on one or more denture libraries, or a size, position, shape, and orientation of the ungrown or incompletely grown tooth based on statistics under big data.

In some embodiments, the first preset parameters include the following parameters of the mesial adjacent teeth: a maximum dimension in a buccolingual diameter direction, a maximum dimension in a mesial-distal direction, and a maximum dimension of a height of a tooth in a long axis direction. The second preset parameters include the following parameters of the distal adjacent teeth: a maximum dimension in the buccolingual diameter direction, a maximum dimension in the mesial-distal direction, and a maximum dimension of the height of the tooth in the long axis direction.

In some specific embodiments, the first preset parameters include the following parameters of the mesial adjacent teeth: a maximum dimension L₁ in the buccolingual diameter direction, a maximum dimension D₁ in the mesial-distal direction and a maximum dimension H₁ of the height of the tooth in the long axis direction. The second preset parameters include the following parameters of the distal adjacent teeth: a maximum dimension L₂ in the buccolingual diameter direction, a maximum dimension D₂ in the mesial-distal direction and a maximum dimension H₂ of the height of the tooth in the long axis direction. In one embodiment, as shown in FIG. 11 and FIG. 12, the eruption cavity 420 on the shell-like dental appliance 400 is provided to encase eruption of a second premolar. At this time, the first preset parameters are a maximum dimension in the buccolingual diameter direction, a maximum dimension in the mesial-distal direction, and a maximum dimension in the height of the tooth in the long axis direction of a first premolar; the second preset parameters are a maximum dimension in the buccolingual diameter direction, a maximum dimension in the mesial-distal direction, and a maximum dimension of the height of the tooth in the long axis direction of a first molar. And the eruption cavity 420 is determined based on the first preset parameters and the second preset parameters is performed by determining the dimension of the eruption cavity 420 in the buccolingual diameter direction by means of arithmetic averaging, weighted averaging, etc. between L1 and L2; determining the dimension of the eruption cavity 420 in the mesial-distal direction by means of arithmetic averaging, weighted averaging, etc. between D1 and D2; determining the dimension of the eruption cavity 420 in the long axis direction of the teeth by means of arithmetic averaging, weighted averaging, etc. between H1 and H2. As a result, the eruption cavity 420 may fit the size and shape of the one or more teeth not grown to the preset eruption parameters throughout the orthodontic plan to the maximum extent.

In some embodiments, the eruption cavity 420 includes a labial/buccal surface, a lingual surface, and an occlusal surface. The labial/buccal surface is a flat surface or a curved surface with a smooth transition to a labial/buccal surface of the mesial adjacent teeth and the distal adjacent teeth, the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth. This setup structure allows the shell-like dental appliance 400 to have a smoother shell-like structure that wraps around the teeth and less foreign body sensation in the mouth when worn by a patient.

In some embodiments, the eruption cavity 420 is also arranged based on a size, a position, a shape, and an orientation of teeth on an opposite jaw corresponding to the one or more teeth not grown to the preset eruption parameters, so that the structure of the eruption cavity 420 does not interfere with maxillomandibular occlusal relationship setting. The occlusal surface of the eruption cavity 420 of this embodiment is designed as a flat, curved or a structure with protrusions and recesses to be matched with opposing teeth. The occlusal surface of the eruption cavity 420 may be designed according to the opposing teeth so that the eruption cavity 420 is cuspid-fossa matching with the opposing teeth, or so that the occlusal surface of this eruption cavity 420 is a structure with protrusions and recesses to be matched with the occlusal surface of the opposing teeth.

### Fifth embodiment

This embodiment provides a method for predicting an eruption cavity of a shell-like dental appliance, in which at least one shell-like dental appliance 500 that gradually adjusts teeth from an initial position to a target orthodontic position while allowing the teeth to erupt according to an orthodontic plan is designed. Herein, one shell-like dental appliance 500 includes a dental appliance body 510 with a geometric structure accommodating multiple maxillary teeth or multiple mandibular teeth and at least one eruption cavity 520 accommodating one or more teeth not grown to the preset eruption parameters. The eruption cavity 520 is predicted based on third preset parameters of distal adjacent teeth and mesial adjacent teeth of the one or more teeth not grown to the preset eruption parameters, such that a gap is set between an inner surface of the eruption cavity 520 and an outer surface of the teeth not grown to the preset eruption parameters. The preset eruption parameters are designed to include parameters of a tooth or teeth formed after full eruption of one or more ungrown or incompletely grown teeth.

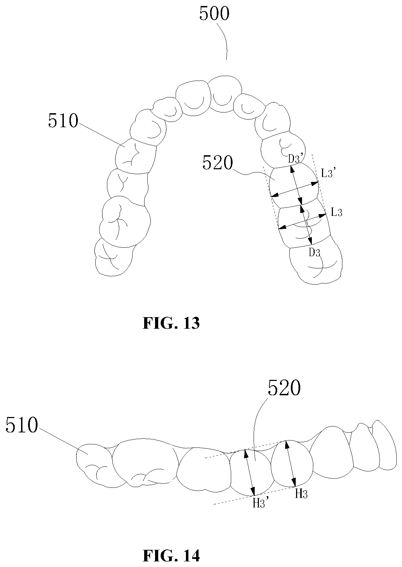

In some embodiments, the third preset parameters include: a maximum dimension in a buccolingual diameter direction, a maximum dimension in a mesial-distal direction of the distal adjacent teeth, and a maximum dimension of a height of a tooth in a long axis direction of the mesial adjacent teeth. In some specific embodiments, as shown in FIG. 13 and FIG. 14, the eruption cavity 520 on the shell-like dental appliance 500 is provided to encase eruption of a second premolar. At this time, the third preset parameters include: a maximum dimension L₃ in a buccolingual diameter direction, a maximum dimension D₃ in a mesial-distal direction, and a maximum dimension H3 of a height in a long axis direction of the mesial adjacent teeth of a first molar, which is used to determine a maximum dimension L₃' in the buccolingual diameter direction, a maximum dimension D₃' in the mesial-distal direction, and a maximum dimension H₃' in the height of the long axis direction of the mesial adjacent teeth of the eruption cavity 520 of the second premolar. The eruption cavity 520 designed based on the maximum dimension in the buccolingual diameter direction of the distal adjacent teeth, the maximum dimension in the mesial-distal direction and the maximum dimension of the height in the long axis direction of the mesial adjacent teeth is sufficient to accommodate the one or more teeth not grown to the preset eruption parameters and such that the dimension of the eruption cavity 520 designed is slightly larger than that of the one or more teeth not grown to the preset eruption parameters, so as to ensure that a gap is set between the eruption cavity 520 and the erupting teeth.

In some embodiments, the eruption cavity 520 includes a labial/buccal surface, a lingual surface, and an occlusal surface. Herein, the labial/buccal surface is a flat surface or a curved surface with a smooth transition to a labial/buccal surface of the mesial adjacent teeth and the distal adjacent teeth; the lingual surface is a flat surface or a curved surface with a smooth transition to a lingual surface of the mesial adjacent teeth and the distal adjacent teeth, and the occlusal surface is a flat surface or a curved surface with a smooth transition to an occlusal surface of the mesial adjacent teeth and the distal adjacent teeth. This setup structure allows the shell-like dental appliance 500 to have a smoother shell-like structure that wraps around the teeth and less foreign body sensation in the mouth when worn by the patient.

In some embodiments, the eruption cavity 520 is further arranged based on a size, a position, a shape, and an orientation of teeth on an opposite jaw corresponding to the one or more teeth not grown to the preset eruption parameters so that the structure of the eruption cavity 520 does not interfere with maxillomandibular occlusal relationship setting. The occlusal surface of the eruption cavity 520 of this embodiment is designed as a flat, curved or a structure with protrusions and recesses to be matched with opposing teeth. The occlusal surface of the eruption cavity 520 is designed based on the opposing teeth so that the eruption cavity 520 is cusp-fossa matching with the opposing teeth, or so that the occlusal surface of the eruption cavity 520 is a structure with protrusions and recesses to be matched with the occlusal surface of the opposing teeth.

In some embodiments, the eruption cavity 520 is a cylindrical structure, an elliptical cylinder, or a multi-prismatic structure with not less than four lateral ribs, which may be set according to a number and type of missing teeth, or may be adaptively selected according to an eruptive gap existing between adjacent teeth.

The disclosed embodiments above are only preferred embodiments of the present application, and the preferred embodiments do not elaborate on all the details. It should be understood that these embodiments are used only to illustrate the present application and not to limit the scope of protection of the present application, which is limited only by the claims.

These embodiments are selected and specifically described in this specification to better explain the principles and practical applications of the present application, so that those skilled in the art can make good use of the present application.

## Claims

1. An orthodontic system comprising a plurality of shell-like dental appliances (100, 200, 400, 500) configured to gradually adjust each of teeth other than unerupted teeth from an initial position to a target orthodontic position according to an orthodontic plan while allowing the unerupted teeth to erupt naturally; wherein each of the plurality of shell-like dental appliances (100, 200, 400, 500) comprises a dental appliance body (110, 210, 410, 510) comprising a geometric structure for accommodating a plurality of maxillary teeth or a plurality of mandibular teeth, the dental appliance body (110, 210, 410, 510) is further provided with at least one eruption portion (120, 220), and a respective eruption portion (120, 220) of the at least one eruption portion (120, 220) is configured to accommodate a respective tooth of one or more teeth not grown to preset eruption parameters; as the orthodontic plan proceeds, eruption portions (120, 220) on the plurality of shell-like dental appliances (100, 200, 400, 500) for accommodating the respective tooth have a constant column structure to be consistent with each other in shape, size, position, and orientation, and the respective eruption portion (120, 220) has an inner surface configured to form a gap between the inner surface and an outer surface of the respective tooth; wherein the preset eruption parameters comprise a size, a position, a shape and an orientation of a tooth or teeth formed after full eruption of one or more ungrown or incompletely grown teeth

2. The orthodontic system according to claim 1, wherein the constant column structure is configured to be arranged based on a size, a position, a shape and an orientation of a tooth or teeth formed after full eruption of the one or more teeth not grown to the preset eruption parameters.

3. The orthodontic system according to claim 2, wherein the column structure is configured to have a size 1.02 to 1.05 times of a size of the tooth or teeth formed after the full eruption of the one or more teeth not grown to the preset eruption parameters; the column structure is configured to be oriented at an angle of 0 to 5 degrees to a long axis of the tooth or teeth formed after the full eruption of the one or more teeth not grown to the preset eruption parameters; the column structure is configured to be positioned at an offset of 0 to 1 mm from a coordinate value in a spatial three-dimensional coordinate system of each of vertices that constitute a position of the tooth or teeth formed after the full eruption of the one or more teeth not grown to the preset eruption parameters; the column structure is configured to be shaped at an offset of 0 to 1 mm from a coordinate value in the spatial three-dimensional coordinate system of each of vertices that constitute a shape of the tooth or teeth formed after the full eruption of the one or more teeth not grown to the preset eruption parameters.

4. The orthodontic system according to claim 2, wherein the constant column structure is further configured to be arranged based on a size, a position, a shape and an orientation of a tooth or teeth on an opposite jaw corresponding to the one or more teeth not grown to the preset eruption parameters, so that the constant column structure does not interfere with a maxillomandibular occlusal relationship.

5. The orthodontic system according to claim 1, wherein the constant column structure is configured to be arranged based on both first preset parameters of mesial adjacent teeth and second preset parameters of distal adjacent teeth of the one or more teeth not grown to the preset eruption parameters.

6. The orthodontic system according to claim 5, wherein the first preset parameters comprise following parameters of the mesial adjacent teeth: a maximum dimension (L1) in a buccolingual diameter direction, a maximum dimension (D1) in a mesial-distal direction, and a maximum dimension (H1) of a height of a tooth in a long axis direction; the second preset parameters comprise the following parameters of the distal adjacent teeth: a maximum dimension (L2) in the buccolingual diameter direction, a maximum dimension (D2) in the mesial-distal direction, and a maximum dimension (H2) of the height of the tooth in the long axis direction.

7. The orthodontic system according to claim 6, wherein the constant column structure comprises a labial/buccal surface, a lingual surface and an occlusal surface, wherein the labial/buccal surface is a flat surface or a curved surface configured to have a smooth transition to a labial/buccal surface of the mesial adjacent teeth and the distal adjacent teeth, the lingual surface is a flat surface or a curved surface configured to have a smooth transition to a lingual surface of the mesial adjacent teeth and the distal adjacent teeth, and the occlusal surface is a flat surface or a curved surface configured to have a smooth transition to an occlusal surface of the mesial adjacent teeth and the distal adjacent teeth.

8. The orthodontic system according to claim 5, wherein the constant column structure is further configured to be arranged based on a size, a position, a shape, and an orientation of teeth on an opposite jaw corresponding to the one or more teeth not grown to the preset eruption parameters, so that the constant column structure does not interfere with a maxillomandibular occlusal relationship.

9. The orthodontic system according to claim 1, wherein the constant column structure is configured to be arranged based on third preset parameters of distal adjacent teeth and mesial adjacent teeth of the one or more teeth not grown to the preset eruption parameters.

10. The orthodontic system according to claim 9, wherein the third preset parameters comprise: a maximum dimension (L3) in a buccolingual diameter direction, a maximum dimension (D3) in a medial-distal direction of the distal adjacent teeth, and a maximum dimension (H3) of a height of a tooth in a long axis direction of the mesial adjacent teeth.

11. The orthodontic system according to claim 9, wherein the constant column structure comprises a labial/buccal surface, a lingual surface and an occlusal surface, wherein the labial/buccal surface is a flat surface or a curved surface configured to have a smooth transition to a labial/buccal surface of the mesial adjacent teeth and the distal adjacent teeth, the lingual surface is a flat surface or a curved surface configured to have a smooth transition to a lingual surface of the mesial adjacent teeth and the distal adjacent teeth, and the occlusal surface is a flat surface or a curved surface configured to have a smooth transition to an occlusal surface of the mesial adjacent teeth and the distal adjacent teeth.

12. The orthodontic system according to claim 9, wherein the constant column structure is further configured to be arranged based on a size, a position, a shape, and an orientation of teeth on an opposite jaw corresponding to the one or more teeth not grown to the preset eruption parameters, so that the constant column structure does not interfere with a maxillomandibular occlusal relationship.

13. The orthodontic system according to claim 1, wherein the constant column structure is a cylindrical structure, an elliptical cylindrical structure, or a multi-prismatic column structure with not less than four lateral ribs.

14. The orthodontic system according to claim 1, wherein the geometric structure on each of the plurality of shell-like dental appliances (100, 200, 400, 500) other than the eruption portion (120, 220) is configured to gradually adjust each of the teeth other than the unerupted teeth from the initial position to the target orthodontic position.

15. A design method of an orthodontic system, wherein the design method comprises:
S1. obtaining a digital dental model: obtaining (S1) the digital dental model, wherein the digital dental model comprises a digital teeth model and a digital gingival model;
S2. segmenting and identifying the digital dental model: segmenting (S2) the digital dental model into the digital gingival model and a plurality of digital tooth crown models in a one-to-one correspondence to teeth; identifying and marking data indicating teeth which do not erupt or do not fully erupt;
S3. virtually designing an orthodontic plan: designing (S3) said plurality of digital tooth crown models virtually so that each of the plurality of digital tooth crown models gradually changes from an initial position to a target orthodontic position to obtain a series of intermediate digital dental models;
S4. designing the orthodontic system:
designing (S4) a plurality of shell-like dental appliances configured to gradually adjust each of the teeth from the initial position to the target orthodontic position according to the orthodontic plan while allowing the unerupted teeth to erupt naturally, wherein each of the plurality of shell-like dental appliances comprises a dental appliance body comprising a geometric structure for accommodating a plurality of maxillary teeth or a plurality of mandibular teeth, the dental appliance body is further provided with at least one eruption portion, and a respective eruption portion of the at least one eruption portion is configured to accommodate a respective tooth of one or more teeth not grown to preset eruption parameters;
as the orthodontic plan proceeds, eruption portions on the plurality of shell-like dental appliances for accommodating the respective tooth have a constant column structure to be consistent with each other in shape, size, position, and orientation, and the respective eruption portion is configured to form a gap between an inner surface and an outer surface of the respective tooth; wherein the preset eruption parameters comprise a size, a position, a shape and an orientation of a tooth or teeth formed after full eruption of one or more ungrown or incompletely grown teeth.

## Patentansprüche

1. Orthodontisches System, umfassend eine Vielzahl von schalenförmigen dentalen Vorrichtungen (100, 200, 400, 500), die dazu konfiguriert sind, allmählich alle anderen Zähne als nicht durchgebrochene Zähne von einer anfänglichen Position an eine angestrebte orthodontische Position gemäß einem orthodontischen Plan anzupassen und es dabei den nicht durchgebrochenen Zähnen zu erlauben, natürlich durchzubrechen:
wobei jede der Vielzahl von schalenförmigen dentalen Vorrichtungen (100, 200, 400, 500) einen dentalen Vorrichtungskörper (110, 210, 410, 510) umfasst, der eine geometrische Struktur zum Unterbringen einer Vielzahl von Oberkieferzähnen oder einer Vielzahl von Unterkieferzähnen umfasst, wobei der dentale Vorrichtungskörper (110, 210, 410, 510) ferner mit mindestens einem Durchbruchabschnitt (120, 220) versehen ist, und ein jeweiliger Durchbruchabschnitt (120, 220) des mindestens einen Durchbruchabschnitts (120, 220) dazu konfiguriert ist, einen jeweiligen Zahn von einem oder mehreren Zähnen, die nicht gemäß voreingestellten Durchbruchparametern gewachsen sind, unterzubringen; wobei, während der orthodontische Plan fortschreitet, die Durchbruchabschnitte (120, 220) an der Vielzahl von schalenförmigen dentalen Vorrichtungen (100, 200, 400, 500) zum Unterbringen des jeweiligen Zahns eine konstante Säulenstruktur aufweisen, um in Form, Größe, Position und Orientation untereinander einheitlich zu sein, und der jeweilige Durchbruchabschnitt (120, 220) eine innere Oberfläche aufweist, die dazu konfiguriert ist, eine Lücke zwischen der inneren Oberfläche und einer äußeren Oberfläche des jeweiligen Zahns zu bilden; wobei die voreingestellten Durchbruchparameter eine Größe, eine Position, eine Form und eine Orientierung eines Zahns oder von Zähnen umfassen, die sich nach einem vollständigen Durchbruch eines oder mehrerer nicht gewachsener oder unvollständig gewachsener Zähne bilden.

2. Orthodontisches System nach Anspruch 1, wobei die konstante Säulenstruktur dazu konfiguriert ist, basierend auf einer Größe, einer Position, einer Form und einer Orientierung eines Zahns oder von Zähnen, die sich nach dem vollständigen Durchbruch des einen Zahns oder der mehreren Zähnen, die nicht gemäß den voreingestellten Durchbruchparametern gewachsen sind, bilden, angeordnet zu sein.

3. Orthodontisches System nach Anspruch 2, wobei die Säulenstruktur dazu konfiguriert ist, eine Größe aufzuweisen, die 1,02 bis 1,05mal die Größe des Zahns oder der Zähne ist, die sich nach dem vollständigen Durchbruch des einen oder der mehreren Zähne, die nicht gemäß den voreingestellten Durchbruchparametern gewachsen sind, bilden; wobei die Säulenstruktur dazu konfiguriert ist, in einem Winkel von 0 bis 5 Grad zu einer Längsachse des Zahns oder der Zähne, die sich nach dem vollständigen Durchbruch des einen oder der mehreren Zähne, die nicht gemäß den voreingestellten Durchbruchparametern gewachsen sind, bilden, orientiert zu sein; wobei die Säulenstruktur dazu konfiguriert ist, mit einem Versatz von 0 bis 1 mm gegenüber einem Koordinatenwert in einem räumlichen dreidimensionalen Koordinatensystem jedes der Scheitel, die eine Position des Zahns oder der Zähne bilden, die sich nach dem vollständigen Durchbruch des einen oder der mehreren Zähne, die nicht gemäß den voreingestellten Durchbruchparametern gewachsen sind, bilden, positioniert zu werden; wobei die Säulenstruktur dazu konfiguriert ist, mit einem Versatz von 0 bis 1 mm gegenüber einem Koordinatenwert in einem räumlichen dreidimensionalen Koordinatensystem jedes der Scheitel, die eine Form des Zahns oder der Zähne bilden, die sich nach dem vollständigen Durchbruch des einen oder der mehreren Zähne, die nicht gemäß den voreingestellten Durchbruchparametern gewachsen sind, bilden, positioniert zu werden.

4. Orthodontisches System nach Anspruch 2, wobei die konstante Säulenstruktur ferner dazu konfiguriert ist, basierend auf einer Größe, einer Position, einer Form und einer Orientierung eines Zahns oder mehrerer Zähne auf einem gegenüberliegenden Kiefer, die dem einen oder den mehreren Zähnen, die nicht gemäß den voreingestellten Durchbruchparametern gewachsen sind, entsprechen, so dass die konstante Säulenstruktur eine maxillomandibulare okklusale Beziehung nicht stört.

5. Orthodontisches System nach Anspruch 1, wobei die konstante Säulenstruktur dazu konfiguriert ist, basierend sowohl auf ersten voreingestellten Parametern von mesialen angrenzenden Zähnen als auch auf zweiten voreingestellten Parametern von distalen angrenzenden Zähnen des einen oder der mehreren Zähne, die nicht gemäß den voreingestellten Durchbruchparametern gewachsen sind, angeordnet zu sein.

6. Orthodontisches System nach Anspruch 5, wobei die ersten voreingestellten Parameter folgende Parameter der mesialen angrenzenden Zähne umfassen: eine maximale Dimension (L1) in einer bukkolingualen Durchmesserrichtung, eine maximale Dimension (D1) in einer mesial-distalen Richtung, und eine maximale Dimension (H1) einer Höhe eines Zahns in einer Längsachsenrichtung; wobei die zweiten voreingestellten Parameter die folgenden Parameter der distalen angrenzenden Zähne umfassen: eine maximale Dimension (L2) in der bukkolingualen Durchmesserrichtung, eine maximale Dimension (D2) in der mesial-distalen Richtung, und eine maximale Dimension (H2) der Höhe des Zahns in der Längsachsenrichtung.

7. Orthodontisches System nach Anspruch 6, wobei die konstante Säulenstruktur eine labiale/bukkale Oberfläche, eine linguale Oberfläche und eine okklusale Oberfläche umfasst, wobei die labiale/bukkale Oberfläche eine flache Oberfläche oder eine gekrümmte Oberfläche ist, die dazu konfiguriert ist, einen nahtlosen Übergang zu einer labialen/bukkalen Oberfläche der mesialen angrenzenden Zähne und der distalen angrenzenden Zähne aufzuweisen, wobei die linguale Oberfläche eine flache Oberfläche oder eine gekrümmte Oberfläche ist, die dazu konfiguriert ist, einen nahtlosen Übergang zu einer lingualen Oberfläche der mesialen angrenzenden Zähne und der distalen angrenzenden Zähne aufzuweisen, und die okklusale Oberfläche eine flache Oberfläche oder eine gekrümmte Oberfläche ist, die dazu konfiguriert ist, einen nahtlosen Übergang zu einer okklusalen Oberfläche der mesialen angrenzenden Zähne und der distalen angrenzenden Zähne aufzuweisen.

8. Orthodontisches System nach Anspruch 5, wobei die konstante Säulenstruktur ferner dazu konfiguriert ist, basierend auf einer Größe, einer Position, einer Form und einer Orientierung von Zähnen auf einem gegenüberliegenden Kiefer angeordnet zu sein, die dem einen oder den mehreren Zähnen entspricht, die nicht gemäß den voreingestellten Durchbruchparametern gewachsen sind, so dass die konstante Säulenstruktur eine maxillomandibulare okklusale Beziehung nicht stört.

9. Orthodontisches System nach Anspruch 1, wobei die konstante Säulenstruktur dazu konfiguriert ist, basierend auf dritten voreingestellten Parametern von distalen angrenzenden Zähnen und mesialen angrenzenden Zähnen des einen oder der mehreren Zähne, die nicht gemäß den voreingestellten Durchbruchparametern gewachsen sind, angeordnet zu sein.

10. Orthodontisches System nach Anspruch 9, wobei die dritten voreingestellten Parameter umfassen: ein maximale Dimension (L3) in einer bukkolingualen Durchmesserrichtung, eine maximale Dimension (D3) in einer medial-distalen Richtung distal angrenzender Zähne, und eine maximale Dimension (H3) einer Höhe eines Zahns in einer Längsachsenrichtung der mesialen angrenzenden Zähne.

11. Orthodontisches System nach Anspruch 9, wobei die konstante Säulenstruktur eine labiale/bukkale Oberfläche, eine linguale Oberfläche und eine okklusale Oberfläche umfasst, wobei die labiale/bukkale Oberfläche eine flache Oberfläche oder eine gekrümmte Oberfläche ist, die dazu konfiguriert ist, einen nahtlosen Übergang zu einer labialen/bukkalen Oberfläche der mesialen angrenzenden Zähne und der distalen angrenzenden Zähne aufzuweisen, wobei die linguale Oberfläche eine flache Oberfläche oder eine gekrümmte Oberfläche ist, die dazu konfiguriert ist, einen nahtlosen Übergang zu einer lingualen Oberfläche der mesialen angrenzenden Zähne und der distalen angrenzenden Zähne aufzuweisen, und die okklusale Oberfläche eine flache Oberfläche oder eine gekrümmte Oberfläche ist, die dazu konfiguriert ist, einen nahtlosen Übergang zu einer okklusalen Oberfläche der mesialen angrenzenden Zähne und der distalen angrenzenden Zähne aufzuweisen.

12. Orthodontisches System nach Anspruch 9, wobei die konstante Säulenstruktur ferner dazu konfiguriert ist, basierend auf einer Größe, einer Position, einer Form und einer Orientierung der Zähne auf einem gegenüberliegenden Kiefer angeordnet zu sein, die dem einen oder den mehreren Zähnen, die nicht gemäß den voreingestellten Durchbruchparametern gewachsen sind, entsprechen, so dass die konstante Säulenstruktur eine maxillomandibulare okklusale Beziehung nicht stört.

13. Orthodontisches System nach Anspruch 1, wobei die konstante Säulenstruktur eine zylindrische Struktur, eine elliptische zylindrische Struktur oder eine multiprismatische Säulenstruktur mit nicht weniger als vier lateralen Rippen ist.

14. Orthodontisches System nach Anspruch 1, wobei die geometrische Struktur auf allen anderen der Vielzahl von schalenförmigen dentalen Vorrichtungen (100, 200, 400, 500) als dem Durchbruchabschnitt (120, 220) dazu konfiguriert ist, allmählich alle anderen Zähne als die nicht durchgebrochenen Zähne von der anfänglichen Position an die angestrebte orthodontische Position anzupassen.

15. Gestaltungsverfahren eines orthodontischen Systems, wobei das Gestaltungsverfahren umfasst:
S1. Erzielen eines digitalen dentalen Modells: Erzielen (S1) des digitalen dentalen Modells, wobei das digitale dentale Modell ein digitales Zahnmodell und ein digitales Zahnfleischmodell umfasst;
S2. Segmentieren und Identifizieren des digitalen dentalen Modells: Segmentieren (S2) des digitalen dentalen Modells in das digitale Zahnfleischmodell und eine Vielzahl von digitalen Zahnkronenmodellen in einer Eins-zu-Eins-Entsprechung zu den Zähnen; Identifizieren und Markieren von Daten, die Zähne angeben, die nicht durchbrechen oder nicht ganz durchbrechen;
S3. virtuelles Gestalten eines orthodontischen Plans: virtuelles Gestalten (S3) der Vielzahl von digitalen Zahnkronenmodellen, so dass jedes der Vielzahl von digitalen Zahnkronenmodellen sich allmählich von einer anfänglichen Position in eine angestrebte orthodontische Position ändert, um eine Reihe von dazwischenliegenden digitalen dentalen Modellen zu erzielen;
S4. Gestalten des orthodontischen Systems:
Gestalten (S4) einer Vielzahl von schalenförmigen dentalen Vorrichtungen, die dazu konfiguriert sind, allmählich jeden der Zähne von der anfänglichen Position an die angestrebte orthodontische Position gemäß dem orthodontischen Plan anzupassen und dabei die nicht durchgebrochenen Zähne natürlich durchbrechen zu lassen, wobei jede der Vielzahl von schalenförmigen dentalen Vorrichtungen einen dentalen Vorrichtungskörper umfasst, der eine geometrische Struktur zum Unterbringen einer Vielzahl von Oberkieferzähnen oder einer Vielzahl von Unterkieferzähnen umfasst, wobei der dentale Vorrichtungskörper ferner mit mindestens einem Durchbruchabschnitt versehen ist, und ein jeweiliger Durchbruchabschnitt des mindestens einen Durchbruchabschnitts dazu konfiguriert ist, einen jeweiligen Zahn von einem oder mehreren Zähnen, die nicht gemäß voreingestellten Durchbruchparametern gewachsen sind, unterzubringen;
wobei, während der orthodontische Plan fortschreitet, Durchbruchabschnitte an der Vielzahl von schalenförmigen dentalen Vorrichtungen zum Unterbringen des jeweiligen Zahns eine konstante Säulenstruktur aufweisen, um in Form, Größe, Position und Orientation untereinander einheitlich zu sein, und der jeweilige Durchbruchabschnitt dazu konfiguriert ist, eine Lücke zwischen einer inneren Oberfläche und einer äußeren Oberfläche des jeweiligen Zahns zu bilden; wobei die voreingestellten Durchbruchparameter eine Größe, eine Position, eine Form und eine Orientierung eines Zahns oder von Zähnen umfassen, die sich nach einem vollständigen Durchbruch eines oder mehrerer nicht gewachsener oder unvollständig gewachsener Zähne bilden.

## Revendications

1. Système orthodontique comprenant une pluralité d'appareils dentaires en forme de coque (100, 200, 400, 500) configurés pour ajuster progressivement chacune des dents autres que les dents incluses d'une position initiale à une position orthodontique cible selon un plan orthodontique tout en permettant aux dents incluses de pousser naturellement ;
dans lequel chacun de la pluralité d'appareils dentaires en forme de coque (100, 200, 400, 500) comprend un corps d'appareil dentaire (110, 210, 410, 510) comprenant une structure géométrique pour loger une pluralité de dents maxillaires ou une pluralité de dents mandibulaires, le corps d'appareil dentaire (110, 210, 410, 510) est en outre prévu avec au moins une partie d'éruption (120, 220) et une partie d'éruption (120, 220) respective de la au moins une partie d'éruption (120, 220) est configurée pour loger une dent respective des une ou plusieurs dents qui ne sont pas encore sorties selon les paramètres d'éruption prédéfinis ; au fur et à mesure de l'exécution du plan orthodontique, les parties d'éruption (120, 220) sur la pluralité d'appareils dentaires en forme de coque (100, 200, 400, 500) pour loger la dent respective ont une structure de colonne constante pour être conformes entre elles du point de vue de la forme, de la taille, de la position et de l'orientation, et la partie d'éruption (120, 220) respective a une surface interne configurée pour former un interstice entre la surface interne et la surface externe de la dent respective ; dans lequel les paramètres d'éruption prédéfinis comprennent une taille, une position, une forme et une orientation d'une dent ou des dents formée(s) après l'éruption complète d'une ou de plusieurs dents incluses ou pas tout à fait sorties.

2. Système orthodontique selon la revendication 1, dans lequel la structure de colonne constante est configurée pour être agencée sur la base d'une taille, d'une position, d'une forme et d'une orientation d'une dent ou des dents formée(s) après éruption complète des une ou plusieurs dents qui ne sont pas encore sorties selon les paramètres d'éruption prédéfinis.

3. Système orthodontique selon la revendication 2, dans lequel la structure de colonne est configurée pour avoir une taille représentant 1,02 à 1,05 fois la taille de la dent ou des dents formée(s) après l'éruption complète des une ou plusieurs dents qui ne sont pas encore sorties selon les paramètres d'éruption prédéfinis ; la structure de colonne est configurée pour être orientée à un angle de 0 à 5 degrés par rapport à un axe long de la dent ou des dents formée(s) après l'éruption complète des une ou plusieurs dents qui ne sont pas encore sorties selon les paramètres d'éruption prédéfinis ; la structure de colonne est configurée pour être positionnée à un décalage de 0 à 1 mm d'une valeur de coordonnée dans un système de coordonnées tridimensionnel dans l'espace de chacun des sommets qui constituent une position de la dent ou des dents formée(s) après l'éruption complète des une ou plusieurs dents qui ne sont pas encore sorties selon les paramètres d'éruption prédéfinis ; la structure de colonne est configurée pour être formée à un décalage de 0 à 1 mm d'une valeur de coordonnée dans le système de coordonnées tridimensionnel dans l'espace de chacun des sommets qui constituent une forme de la dent ou des dents formée(s) après l'éruption complète des une ou plusieurs dents qui ne sont pas encore sorties selon les paramètres d'éruption prédéfinis.

4. Système orthodontique selon la revendication 2, dans lequel la structure de colonne constante est en outre configurée pour être agencée sur la base d'une taille, d'une position, d'une forme et d'une orientation d'une dent ou des dents sur une mâchoire opposée correspondant aux une ou plusieurs dents qui ne sont pas encore sorties selon les paramètres d'éruption prédéfinis, de sorte que la structure de colonne constante n'interfère pas avec une relation maxillo-mandibulaire occlusale.

5. Système orthodontique selon la revendication 1, dans lequel la structure de colonne constante est configurée pour être agencée sur la base à la fois des premiers paramètres prédéfinis des dents mésiales adjacentes et des deuxièmes paramètres prédéfinis des dents distales adjacentes des une ou plusieurs dents qui ne sont pas encore sorties selon les paramètres d'éruption prédéfinis.

6. Système orthodontique selon la revendication 5, dans lequel les premiers paramètres prédéfinis comprennent les paramètres suivants des dents mésiales adjacentes : une dimension maximum (L1) dans une direction de diamètre buccolingual, une dimension maximum (D1) dans une direction mésiale - distale, et une dimension maximum (H1) d'une hauteur d'une dent dans une direction d'axe long ; les deuxièmes paramètres prédéfinis comprennent les paramètres suivants des dents distales adjacentes : une dimension maximum (L2) dans la direction de diamètre buccolingual, une dimension maximum (D2) dans la direction mésiale - distale, et une dimension maximum (H2) de la hauteur de la dent dans la direction d'axe long.

7. Système orthodontique selon la revendication 6, dans lequel la structure de colonne constante comprend une surface labiale/buccale, une surface linguale et une surface occlusale, dans lequel la surface labiale/buccale est une surface plate ou une surface courbée configurée pour avoir une transition en douceur avec une surface labiale/buccale des dents mésiales adjacentes et des dents distales adjacentes, la surface linguale est une surface plate ou une surface courbée configurée pour avoir une transition en douceur avec une surface linguale des dents mésiales adjacentes et des dents distales adjacentes, et la surface occlusale est une surface plate ou une surface courbée configurée pour avoir une transition en douceur avec une surface occlusale des dents mésiales adjacentes et des dents distales adjacentes.

8. Système orthodontique selon la revendication 5, dans lequel la structure de colonne constante est en outre configurée pour être agencée sur la base d'une taille, d'une position, d'une forme et d'une orientation des dents sur une mâchoire opposée correspondant aux une ou plusieurs dents qui ne sont pas encore sorties selon les paramètres d'éruption prédéfinis, de sorte que la structure de colonne constante n'interfère pas avec une relation maxillo-mandibulaire occlusale.

9. Système orthodontique selon la revendication 1, dans lequel la structure de colonne constante est configurée pour être agencée sur la base de troisièmes paramètres prédéfinis des dents distales adjacentes et des dents mésiales adjacentes des une ou plusieurs dents qui ne sont pas encore sorties selon les paramètres d'éruption prédéfinis.

10. Système orthodontique selon la revendication 9, dans lequel les troisièmes paramètres prédéfinis comprennent : une dimension maximum (L3) dans une direction de diamètre buccolingual, une dimension maximum (D3) dans une direction médiale-distale des dents distales adjacentes, et une dimension maximum (H3) d'une hauteur d'une dent dans une direction d'axe long des dents mésiales adjacentes.

11. Système orthodontique selon la revendication 9, dans lequel la structure de colonne constante comprend une surface labiale/buccale, une surface linguale et une surface occlusale, dans lequel la surface labiale/buccale est une surface plate ou une surface courbée configurée pour avoir une transition en douceur avec une surface labiale/buccale des dents mésiales adjacentes et des dents distales adjacentes, la surface linguale est une surface plate ou une surface courbée configurée pour avoir une transition en douceur avec une surface linguale des dents mésiales adjacentes et des dents distales adjacentes, et la surface occlusale est une surface plate ou une surface courbée configurée pour avoir une transition en douceur avec une surface occlusale des dents mésiales adjacentes et des dents distales adjacentes.

12. Système orthodontique selon la revendication 9, dans lequel la structure de colonne constante est en outre configurée pour être agencée sur la base d'une taille, d'une position, d'une forme et d'une orientation des dents sur une mâchoire opposée correspondant aux une ou plusieurs dents qui ne sont pas encore sorties selon les paramètres d'éruption prédéfinis, de sorte que la structure de colonne constante n'interfère pas avec une relation maxillo-mandibulaire occlusale.

13. Système orthodontique selon la revendication 1, dans lequel la structure de colonne constante est une structure cylindrique, une structure cylindrique elliptique ou une structure de colonne à plusieurs prismes avec pas moins de quatre nervures latérales.

14. Système orthodontique selon la revendication 1, dans lequel la structure géométrique sur chacun de la pluralité d'appareils dentaires en forme de coque (100, 200, 400, 500) autres que la partie d'éruption (120, 220) est configurée pour ajuster progressivement chacune des dents autres que les dents incluses de la position initiale à la position orthodontique cible.

15. Procédé de conception d'un système orthodontique, dans lequel le procédé de conception comprend les étapes consistant à :
S1. obtenir un modèle dentaire numérique : obtenir (S1) le modèle dentaire numérique, dans lequel le modèle dentaire numérique comprend un modèle de dents numérique et un modèle gingival numérique ;
S2. segmenter et identifier le modèle dentaire numérique : segmenter (S2) le modèle dentaire numérique en modèle gingival numérique et une pluralité de modèles de couronne de dent numériques en une correspondance une à une par rapport aux dents ; identifier et marquer des données indiquant les dents qui sont incluses ou ne sont pas complètement sorties ;
S3. concevoir virtuellement un plan orthodontique : concevoir (S3) ladite pluralité de modèles de couronne de dent numériques virtuellement de sorte que chacun de la pluralité de modèles de couronne de dent numériques passe progressivement d'une position initiale à une position orthodontique cible pour obtenir une série de modèles dentaires numériques intermédiaires ;
S4. concevoir le système orthodontique :
concevoir (S4) une pluralité d'appareils dentaires en forme de coque configurés pour ajuster progressivement chacune des dents de la position initiale à la position orthodontique cible selon le plan orthodontique tout en permettant aux dents incluses de pousser naturellement, dans lequel chacun de la pluralité d'appareils dentaires en forme de coque comprend un corps d'appareil dentaire comprenant une structure géométrique pour loger une pluralité de dents maxillaires ou une pluralité de dents mandibulaires, le corps d'appareil dentaire est en outre prévu avec au moins une partie d'éruption, et une partie d'éruption respective de la au moins une partie d'éruption est configurée pour loger une dent respective des une ou plusieurs dents qui ne sont pas encore sorties selon les paramètres d'éruption prédéfinis ;
au fur et à mesure de l'exécution du plan orthodontique, les parties d'éruption sur la pluralité d'appareils dentaires en forme de coque pour loger la dent respective ont une structure de colonne constante pour être conformes entre elles du point de vue de la forme, de la taille, de la position et de l'orientation, et la partie d'éruption respective est configurée pour former un interstice entre la surface interne et une surface externe de la dent respective ; dans lequel les paramètres d'éruption prédéfinis comprennent une taille, une position, une forme et une orientation d'une dent ou des dents formée(s) après l'éruption complète d'une ou de plusieurs dents incluses ou pas tout à fait sorties.
